# EUROPEAN PATENT APPLICATION

(11) **EP 1 955 671 A1**
(43) Date of publication of application: **13.08.2008**
(21) Application number: 07102091.1
(22) Date of filing: 09.02.2007
(51) Int. Cl.: A61C 11/02, A61C 11/08, A61C 13/00

(54) **3-D orthognathic surgery simulation**

(71) Applicant: Universiteit Antwerpen, 2000 Antwerpen (BE)
(72) Inventor: Okkerse, Walter, 2520 Emblem (BE); Okkerse, Bert, 2560 Nijlen (BE); Brys, Egied, 2870 Liesele (BE); Brebels, Adriaan, 2290 Vorselaar (BE); Verbist, Paul, 2540 Hove (BE)
(74) Representative: Quintelier, Claude

(57) **Abstract**

The invention relates to a method for simulating an orthognathic operation, wherein, to simulate the displacement of the upper dental arch (105) of the patient caused by the surgical intervention to be applied to the maxilla (106) during said orthognathic surgery, the angular and translational position of an upper mounting plate (526) of a 3-D orthognathic surgery simulator (510) carrying a model (317) of said upper dental arch (105) is readjusted by rotating said upper mounting plate (526) around at least two, preferably three of three orthogonal axes X,Y and Z with respect to a rotation point (522) fixed to an arm (521) of said 3-D orthognathic surgery simulator (510), and translating said arm (521) substantially parallel to at least one of the same three orthogonal axes X,Y,Z.

## Description

The present invention concerns a method of simulating an orthognathic surgery operation, a device for determining a new spatial position, that is, angle and position, of an upper dental arch with respect to a skull reference frame, so as to be able to transpose the results of said simulation method to the operation, and a method of using said device.

The primary purpose of orthognathic surgery is to correct malocclusions, that is, misalignments between the upper and lower dental arches of a patient, such as prognathism (underbite) or retrognathism (overbite) open bite and mandibular laterodeviation. If desireable, also aesthetic corrections can be carried out.

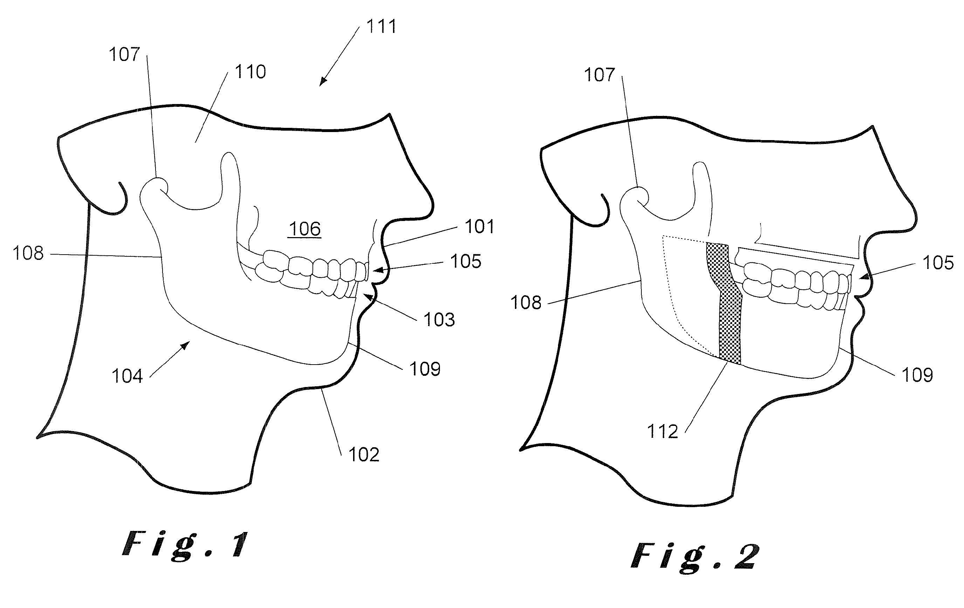

Fig. 1 represents the upper and lower jaws 101,102 of a patient with malocclusion, in this case retrognathism. The lower dental arch 103 in the mandible 104 is therefore in retreat with respect to the upper dental arch 105 in the maxilla 106.

The mandible 104 comprises the condyles 107, the rami 108 and the mandibular body 109. Each condyle 107 is hinged to the temporal bone 110 at one side of the skull 111 to form the temporomandibular joint, and is connected by a ramus 108 to the mandibular body 109, which carries the lower dental arch 103. It must be noted that said temporomandibular joint is not a simple hinge joint, but permits additional movements. In particular, when the mouth is opened widely, the rotation of the condyles 107 is accompanied by a forward movement. This is the only place in the human body where one bone has two junctions.

Fig. 2 illustrates the upper and lower jaws 101,102 of the same patient after orthognathic surgery. The maxilla 106 is split and rejoined so as to correct, for example, the alignment of the middle line of the upper dental arch 105, and the angle of the upper dental arch 105. Each ramus 108 is split in a plane 112, to separate the mandibular body 109 from the condyles 107 and rejoined after displacing the mandibular body 109 relatively to the ramus 108 so as to correct the over- or underbite.

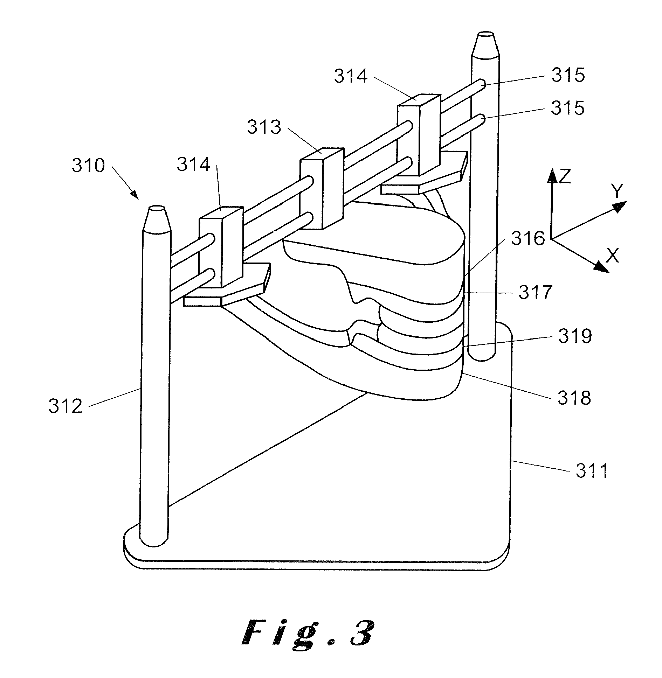

Before orthognathic surgery, it is customary to perform a simulation of the necessary interventions at the maxilla 106 and the mandible 104. For this simulation, an adjustable articulator 310 is conventionally used, such as schematically depicted in Fig. 3. This illustrated adjustable articulator 310 is mounted on a base 311 and comprises a frame 312, a maxillar support 313 and two condylar supports 314. The frame 312 comprises two parallel bars 315, calibrated with distance markings, for supporting said maxillar and condylar supports 313, 314 in determined positions along an axis Y. An upper mounting plate 316 for supporting an upper dental arch cast 317 is coupled to the frame 312 in a predetermined position in the axes X and Z. The condylar supports 314 comprise articulations adjustable so as to reproduce the temporomandibular joints of the patient. Measurements at the patient permit to determine said positions in the axes X, Y and Z, as well as the rotation axis and the movement of the condyles 107 when opening and closing the mouth, so as to adjust the maxillar and condylar supports 313, 314 and the position of the upper dental arch cast 317 accordingly. A physical mandible model 318 carrying a lower dental arch cast 319 is hung from the condylar supports 314. The mandible model 318 can be made from resin using X-ray images and measurements of the lower jaw 102 of the patient or a plaster model can be made. The occlusion between the lower and upper dental arch casts 317, 319 is checked using an initial bite mould taken from the patient.

With this setup it is therefore possible to simulate the surgical interventions at the maxilla 106 and mandibular body 109. However, this method of simulation has several significant drawbacks.

One problem that can arise in the operation is that, after splitting and rejoining the mandible 104 to correct the occlusion, the mandibular body 109 may protrude below the remi 110. Since the opening angle of the mandible 104 is determined by the temporomandibular joint between the temporal bones 108 and the condyles 107, this can prevent the patient from correctly closing the mouth, leaving him in a permanently slack-jawed position.

Another problem that can arise in the operation is that, after splitting and rejoining the mandible 104 to correct the dental occlusion, the condyles 107 may be asymmetrically displaced in the Y axis. This will leave the patient with mandibular laterodeviations.

Although the downwards position of the mandibular body 109 and the asymmetrical displacement of the condyles 107 can be corrected, respectively, by posterior impacting the maxilla 106, as illustrated, and by rotating it around a vertical axis, it is very difficult to measure exactly these interventions at the maxilla 106 with the adjustable articulators of the prior art and to transpose these measurements to the patient in, as the adjustable articulators of the prior art do not provide adequate means to reproduce those displacements of the maxilla 106, nor are there adequate instruments for transferring them to the patient. As a result, the above-mentioned defects may not be found until after the real surgery, leading to an expensive, complicated and traumatic second operation.

Furthermore, if the amount of bone to be removed from the maxilla 106 in the distal impaction is not measured with great precision, the two fragments of maxilla 106 may set inadequately, with fibrous tissue forming between the two fragments and leading to a pseudo-arthrosis.

United States Patent US 6,102,698 disclosed a method of simulating an orthognatic surgery operation comprising a surgical intervention on the maxilla of a skull for adjusting the position of the upper dental arch in said skull, wherein in said method a 3D orthognatic surgery simulator is used, said simulator comprising an upper mounting plate for mounting a model, preferably a cast, of an upper dental arch, said upper mounting plate of the simulator being mounted on an arm;
wherein said simulation method comprises the steps of:
a) mounting said upper dental arch model on the upper mounting plate of the simulator;
b) adjusting the angular and translational position of the upper mounting plate so as to place the upper dental arch model in a first position representative of the initial position of said upper dental arch with respect to a fixed point in the skull; and
c) readjusting the angular and translational position of the upper mounting plate so as to simulate the surgical intervention to be applied to the maxilla during the orthognathic surgery

However, this prior art has the drawback that the rotational displacements of the upper dental arch model are not adequately measured. Moreover, as the rotation axes are not orthogonal, converting any measurements to a fixed reference system will be very complicated.

The present invention therefore addresses the problem of simulating with great precision a maxillar intervention in orthognathic surgery, including the rotational displacements of the upper dental arch and easily and exactly transposing the results of this simulation to the patient during the operation.

In order to solve this problem, the angular and translational position of said upper mounting plate of the simulator is readjusted by rotating said upper mounting plate around at least two, preferably three of three orthogonal axes X,Y and Z with respect to a rotation point fixed to said arm, and translating said arm substantially parallel to at least one of the same three orthogonal axes X,Y,Z; said simulator being preferably connectable to a computer for controlling rotational and translational displacements of said upper mounting plate of the simulator. Preferably, the simulator is connectable to a computer for controlling rotational and translational displacements of said upper mounting plate of the simulator. This has the advantages of increasing the precision with which the maxillar intervention is simulated, while facilitating the application of complex orthognathic rules to the simulation.

For transposing the results of the simulation more easily to the patient, this method preferably further comprises the steps of taking a first mould of the upper dental arch model in said first position and taking a second mould of the upper dental arch model after readjusting the angular and translational position of the upper mounting plate.

Preferably, the step of taking the first mould comprises the steps of:
a) providing a first teeth indexing member holding a malleable material, preferably acrylic resin, for taking said mould;
b) fixing said first teeth indexing member in a determined position relative to the simulator; and
c) pressing said malleable material against the upper dental arch model;
   and the step of taking the second mould comprises the steps of:
d) providing a second teeth indexing member holding a malleable material, preferably acrylic resin, for taking said mould;
e) fixing said second teeth indexing member in the same determined position relative to the simulator; and
f) pressing said malleable material against the upper dental arch model.

This embodiment has the advantage of allowing the easy creation of first and second moulds of the upper dental arch model before and after the simulated operation with reference to a fixed reference frame so as to correctly transpose the results of the simulation to the patient during the operation.

For this, in a particularly advantageous form, each one of said first and second teeth indexing members comprise a graduated rod, preferably with a half-circular cross-section over at least part of its length, for fixing the teeth indexing member in a determined position and an arch for holding said malleable material.

When said orthognathic surgery operation also comprises an adjustment of a lower dental arch with respect to the condyles in the mandible of said skull, so as to adjust the occlusion between said upper and lower dental arches, it is advantageous if said the method of simulating the operation further comprises the steps of :
a) providing a model, preferably a cast, of the lower dental arch;
b) providing a model of the mandible with condyles, rami, and mandibular body with lower dental arch;
c) simulating in the mandible model the surgical intervention necessary to achieve the desired occlusion; and
d) checking the occlusion between the lower dental arch in the mandible model and a model of the upper dental arch.

This embodiment has the advantage of accurately simulating orthognathic operations comprising interventions both to the maxilla and to the mandible in order to precisely adjust the displacements of the upper and lower dental arches both relative to each other and relative to the cranium.

In a particular embodiment, said mandible model is a physical model, preferably formed at least partly in resin based on X-ray images of the mandible or a plaster model. Such a physical embodiment allows the user to physically appreciate and intervene in the simulation, providing a hands-on feeling much appreciated by surgery professionals.

For this particular embodiment, it is preferable if the simulation method further comprises the step of hanging said mandible model on an adjustable articulated frame suitable for being mounted in said simulator for reproducing the jaw hinge of the patient. This allows a better appreciation of the interactions between the intervention on the mandible and the intervention on the maxilla in a combined device.

For this particular embodiment, a particularly simple and effective way of checking the occlusion comprises the following steps:
a) providing a bite mould of the desired occlusion; and
b) placing, in said simulator, said bite mould of the desired occlusion between said upper dental arch model and said lower dental arch model.

With these steps, it is possible to check the occlusion between the upper and lower dental arch models simply, yet with great accuracy.

In an alternative embodiment, said mandible model is a virtual model. This provides a means of simulating the intervention to the mandible with greater accuracy than could be done on a physical model.

In this alternative embodiment, it is particularly advantageous if the method further comprises the step of providing a virtual model of the skull, comprising at least the maxilla with the upper dental arch, wherein the displacement of the upper dental arch model in the simulator can be reflected in said virtual model of the skull. With this, the relative displacement of the upper and lower dental arches can be visualized in great detail using the virtual models of the skull and mandible.

It is particularly advantageous if said method further comprises the step of taking a CT-scan of the skull, wherein said virtual model of the skull is based on said CT-scan of the skull. This provides a rapidly accessible and accurate source of three-dimensional data of the skull and facilitates the creation of said virtual model of the skull, ensuring its accuracy and completeness.

It is even more advantageous if the CT-scan of the skull is taken with a piece of malleable material, comprising radio-opaque references, held between the upper and lower dental arches, so as to form a bite mould of the initial occlusion, and the method further comprises the following steps:
a) providing models of the upper and lower dental arches;
c) placing these models of the upper and lower dental arches in a desired occlusion with a piece of malleable material, also comprising radio-opaque references, between them, so as to form a bite mould of the desired occlusion; and
c) taking a CT-scan of said models of the upper and lower dental arch in the desired occlusion, with the bite mould of the desired occlusion, for preparing the step of simulating the surgical intervention in the mandible model.

By this process, CT-scans representing the initial and final positions of the upper and lower dental arches are provided that can be used in the virtual models as well as to control the displacements in the 3D-orthognathic surgery simulator.

Even more preferably, this process also comprises a step of taking a CT-scan of said models of the upper and lower dental arch in the initial occlusion, with the bite mould of the initial occlusion. This provides an additional CT-scan representing the initial positions of the upper and lower dental arches without the possible interferences by metallic objects in the oral cavity, such as dental fillings.

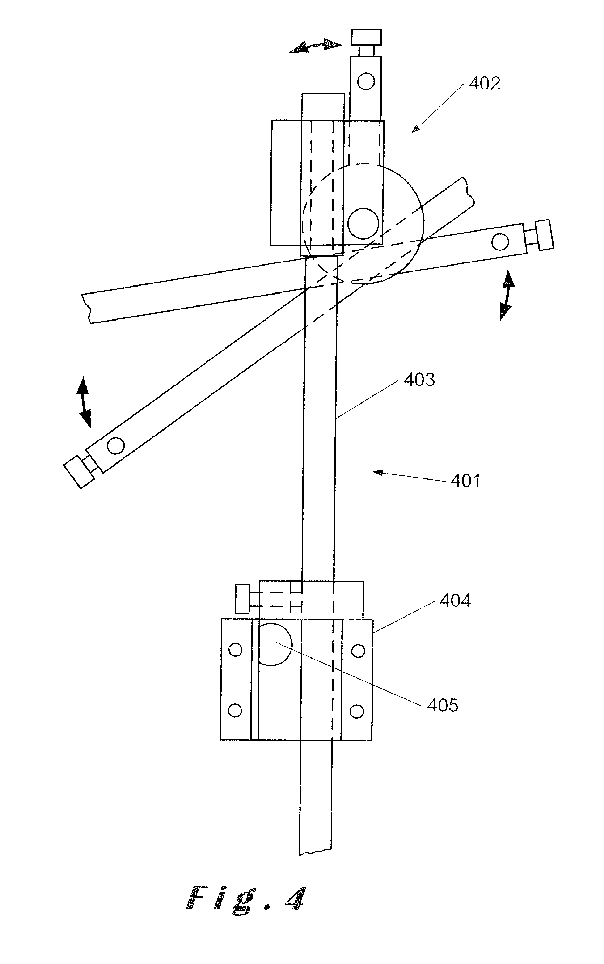

After the simulation, its results need to be transposed to the patient during the operation. This notably means checking whether the displacement of the upper dental arch is the same as in the simulation. The prior art devices and methods for checking the displacement of the upper dental arch from the initial to the desired position in the patient have some additional drawbacks. Fig. 4 represents one such prior art device 401 for determining an angle and position of an upper dental arch 105 with respect to a skull 111 comprise a cranial anchoring section 402 for releasably anchoring the device 401 in a stable position with respect to the skull 111 a first calibrated rod 403 and coupling means 404 for releasably locking said first calibrated rod 403 and a teeth indexing member 405 at a fixed angle and position.

To check the displacement of the upper dental arch 105 from an initial to a desired position using this prior art device, a first teeth indexing member 405 holding a mould 406 of the upper dental arch 105 at the initial position and angle is coupled to this device at a determined position. Then, the device 401 is anchored in a stable position with respect to the skull 111 in which the upper dental arch mould 406 is matched with the upper dental arch 105 in the initial position and angle. The first teeth indexing member 405 is then removed from the device 801 and, after the upper dental arch 105 has been displaced, a second teeth indexing member 405 holding a mould 406 of the upper dental arch 105 at the desired position is coupled to the device in the same determined position, so as to check whether the upper dental arch 105 is now in the desired position.

This device and method have the drawback that, as the device needs to be substantially rigid in order to provide a stable reference, anchoring it to the skull and handling it afterwards is extremely awkward.

The invention therefore also relates to a device for accurately determining an angle and position of an upper dental arch with respect to a skull that can more easily be used to transpose the results of the simulation to the patient.

For this, this device comprises an articulated member for coupling the cranial anchoring section to the first calibrated rod and a first ball joint for coupling the articulated member to the cranial anchoring section, a second ball joint for coupling the articulated member to the first calibrated rod and an articulated joint interposed between the first and the second ball joints, wherein the first and second ball joints and the articulated joint are provided be releasably locked at fixed angles.

The articulated member and first and second ball joints allow easier handling of the device prior to matching the upper dental arch to the mould of the upper dental arch in the initial position and angle. Being releasably lockable at fixed angles, they can however provide a stable reference once the upper dental arch is matched to the mould of the upper dental arch in the initial position and angle.

For further ease of handling, it is preferable if the first and second ball joints and the articulated joint of said articulated member are provided to be simultaneously locked and/or released with a single lever.

In a particularly advantageous embodiment, said cranial anchoring section comprises at least three adjustable attachment means, each one of them comprising:
a) an externally threaded spindle with a claw at one end for releasably anchoring said spindle to one of said bolts; and
b) a rod, preferably having a semicircular cross-section over at least part of its length, and comprising an internally threaded orifice substantially perpendicular to its main axis for receiving said spindle, and a means for releasably locking said spindle in position;
wherein said cranial anchoring section also comprises coupling means for releasably locking said rods of the adjustable attachment means at fixed angles and positions with respect to each other and to the first ball joint of said articulated member. This arrangement facilitates the anchoring of the device to the skull and adds several possibilities for adjusting its position.

For further ease of handling, it is preferable if said rods of the adjustable attachment means are provided to be simultaneously locked and/or released with a single lever.

The invention will be described in detail and non-limitingly with reference to the accompanying figures, in which:
Fig. 5 is a schematic view of a 3D-orthognathic surgery simulator for performing the simulation method of the invention; and
Fig. 6 is a perspective view of an embodiment of the device for accurately determining an angle and position of an upper dental arch with respect to a skull reference frame according to the invention.

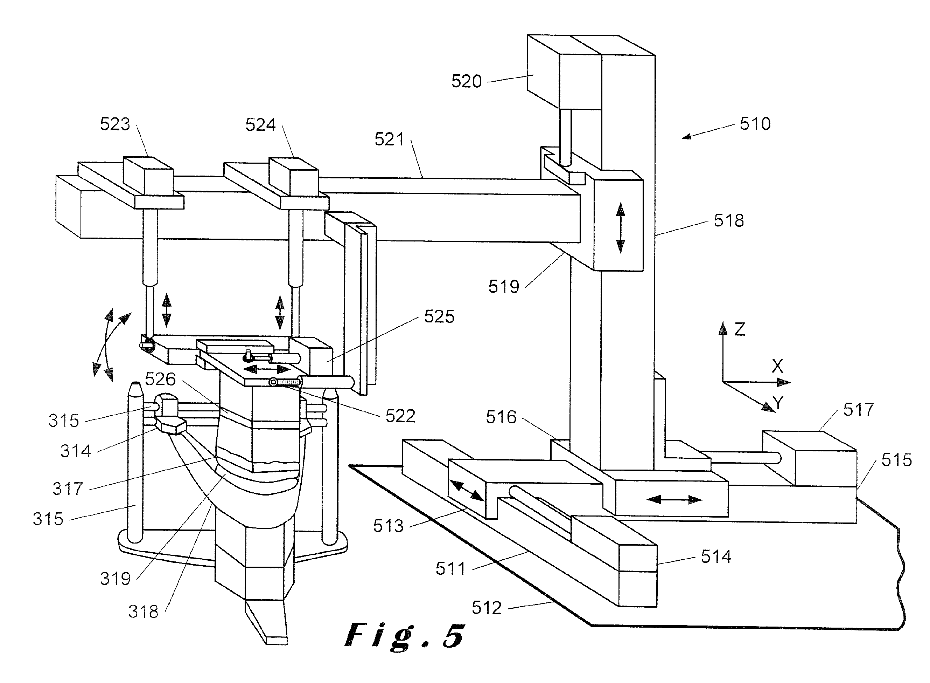

The simulation method of the invention can be carried out using a 3D-orthognathic surgery simulator 510 as illustrated in Fig. 5. The 3D-orthognathic surgery simulator 510 comprises a first rail 511, mounted on a base 512, aligned with a horizontal axis Y and supporting a first chariot 513. A first linear actuator 514 commands the linear motion of said first chariot 513 along said first rail 511. On the first chariot 513 a second rail 515 is mounted aligned with an axis X, also horizontal, but orthogonal to the axis Y. The second rail 515 supports a second chariot 516 and a second linear actuator 517 commands the linear motion of the second chariot 516 along the second rail 515. A third rail 518 is in turn mounted on the second chariot 516. The third rail 518 is aligned with a vertical axis Z and supports a third chariot 519. A third linear actuator 520 commands the linear motion of the third chariot 519 along the third rail 518.

The third chariot 519 holds an arm 521 holding a pivot joint 522, and a fourth, a fifth and a sixth linear actuators 523, 524 and 525. An upper mounting plate 526 for holding an upper dental arch cast 317 is coupled to the arm 521 over said pivot joint 522, and fourth, fifth and sixth linear actuators 523, 524 and 525, so that it can be rotated with respect to said pivot joint around all three orthogonal axes X, Y and Z by differential actuation of the four, fifth and sixth linear actuators 523, 524 and 525.

All six linear actuators 514,517,520,523,524 and 525 are powered by stepping motors that can be controlled by a computer. It is therefore possible to command and record three-dimensional translational and rotational displacements of the upper dental arch cast 317 with respect to a fixed reference with a very high degree of accuracy. It is therefore possible to simulate with this device the precise adjustments of the upper dental arch 105 in the maxilla 106 necessary to correct a given malocclusion.

To prepare the simulation of the operation, first of all the initial state of the upper and lower jaws of the patient has to be determined. Casts 317 and 319 of, respectively, the upper and lower dental arches 105, 103 are taken, as well as an initial bite mould, taken in a relaxed state of the jaw muscles. Further functional and aesthetic measurements can be taken, including:
- The distance that the cutting edge of the maxillary incisors protrudes under the upper lip.
- Whether the gums of the incisors are visible when smiling and by how much.
- Frontal measurements, for example using digital imaging, concerning for example the position of the nose septum and the form of the zygomatic bones.
- The position of the maxilla 106 with respect to the ear channel, determined for example using a face-bow.
- The position of the hinge axis of the temporomandibular joint.
- The inclination of the lower edge of the mandibular body 109.
- The thickness of the skull 111, determined for example using a CT-scan of said skull 111.
- The position of the condyles 107 in the temporomandibular joint.

Using the upper and lower dental arch casts 317, 319, one or several of these measurements, front and side teleradiographies, and/or a CT scan of at least part of the skull 111, it is then possible to construct a model of the mandible 104.

In a first embodiment, said mandible model is a physical mandible model 318, formed for example using resin in combination with the lower dental arch cast 319. This physical mandible model 318 is then hung on a frame 312, for example using articulated condylar supports 314 mounted on substantially parallel bars 315 calibrated with distance markings, as in conventional articulators of the prior art. The frame 312 is then mounted in the 3D-orthognathic surgery simulator 510.

The upper dental arch cast 317 is mounted to the upper mounting plate 526. While in the illustrated embodiment a cast 317 of the upper dental arch 105 is used, any other accurate enough model of the upper dental arch 105, for example a stereolithographic model obtained from a CT-scan, could be used instead. The position of the upper dental arch cast 317 can then be adjusted relative to the physical mandible model 318 in the frame 312 using at least some of the six linear actuators 514,517,520,523,524 and 525 in order to reproduce the initial state of the upper and lower jaws of the patient as determined above. To check whether the occlusion between the upper dental arch cast 317 and the lower dental arch cast 319 correctly reproduces the initial occlusion at the patient, the initial bite mould 320 can be placed between the upper dental arch cast 317 and the lower dental arch cast 319.

Once the initial situation is correctly reproduced in the 3D-orthognathic surgery simulator 510, the frame 312 can be removed from the 3D-orthognathic surgery simulator 510, and a first teeth indexing member 405, comprising in this embodiment a graduated rod and an arch carrying acrylic resin is fixed to a support facing the upper dental arch cast 317 in a determined position. The arch is then pushed against the upper dental arch cast 317, impressing into the acrylic resin the form of the upper dental arch 105 in the original position, so as to form a first mould once the acrylic resin hardens.

Once the first mould is formed, the upper dental arch cast 317 can be removed from the upper mounting plate 526 and used, together with the lower dental arch cast 319, to form a desired bite mould. The upper and lower dental arch casts 317,319 are placed in the desired occlusion with an acrylic resin between them. To hold them together, elastic bands can be attached to bolts placed on both dental arch casts 317,319. When the acrylic resin hardens, it will then form a desired bite mould corresponding to the desired occlusion.

The frame 312 carrying the physical mandible model 318 can then be mounted back into the 3D-orthognathic surgery simulator 510, and the simulation of the operation carried out.

The intervention to the mandible 104 is simulated on the physical mandible model 318, by cutting it at the reproduction of the rami 108 and rejoining it after displacing the reproduction of the mandibular body 109 with respect to the reproduction of the rami 108 so that the lower dental arch cast 319 in the physical mandible model 318 fits against the upper dental arch cast 317 with the desired bite mould placed therebetween. At this point it can be checked whether the reproduction of the mandibular body 109 protrudes below the reproduction of the rami 108, or whether the condylar supports 314 are asymmetrically displaced with respect to their initial positions. In either case, the interventions to the maxilla 106, such as distal impaction or rotation around a substantially vertical axis, which will be required to correct those defects, can then be simulated by displacing the upper dental arch cast 317 with at least one of the six linear actuators 514,517,520,523,524 and 525. Those displacements can be accurately recorded, so as to be used later in the operation to remove the precise amount of maxillar bone and impart an exact displacement to the upper dental arch 105.

The frame 312 carrying the physical mandible model 318 can then be removed again, and a second teeth indexing member 405, also comprising in this embodiment a graduated rod and an arch carrying acrylic resin is fixed to the support facing the upper dental arch cast 317 in the same determined position as the first teeth indexing member 405 when the first mould was taken. A second mould is then taken in the same way as the first mould.

In an alternative embodiment, said mandible model is a virtual mandible model. This virtual mandible model can be manipulated in a computer simulation of the operation.

For this alternative embodiment, a CT-scan of the skull 111 is taken while the patient holds the initial bite mould between his upper and lower dental arches 105, 103. In this embodiment the initial bite mould comprises radio-opaque references embedded in the acrylic resin. Then, as in the first embodiment, a desired bite mould is created by placing the upper and lower dental arch casts 317,319 in the desired occlusion with an acrylic resin between them. In this alternative embodiment, the desired bite mould also comprises radio-opaque references embedded in the acrylic resin. A CT-scan is then taken of the upper and lower dental arch casts 317,319 in the desired occlusion with the desired bite mould between them.

To simulate the operation, the CT-scan of the skull 111 can then be used as an initial state and the CT-scan of the and lower dental arch casts 317,319 in the desired occlusion. The virtual mandible model can be manipulated to correct the occlusion, and a virtual upper dental arch model displaced so as to adjust for an eventual jaw asymmetry or lower protrusion of the mandibular body 109. The displacements of the virtual upper dental arch model are transmited from the computer simulation to the upper dental arch cast 317 mounted on the 3D-orthognathic surgery simulator 510. The upper dental arch cast 317 mounted on the 3D-orthognathic surgery simulator 510 can therefore be used as a physical reference, from which the first mould, representing the initial position of the upper dental arch 105, and the second mould, representing the desired position of the upper dental arch 105 after the operation, can be taken using the method described for the first embodiment.

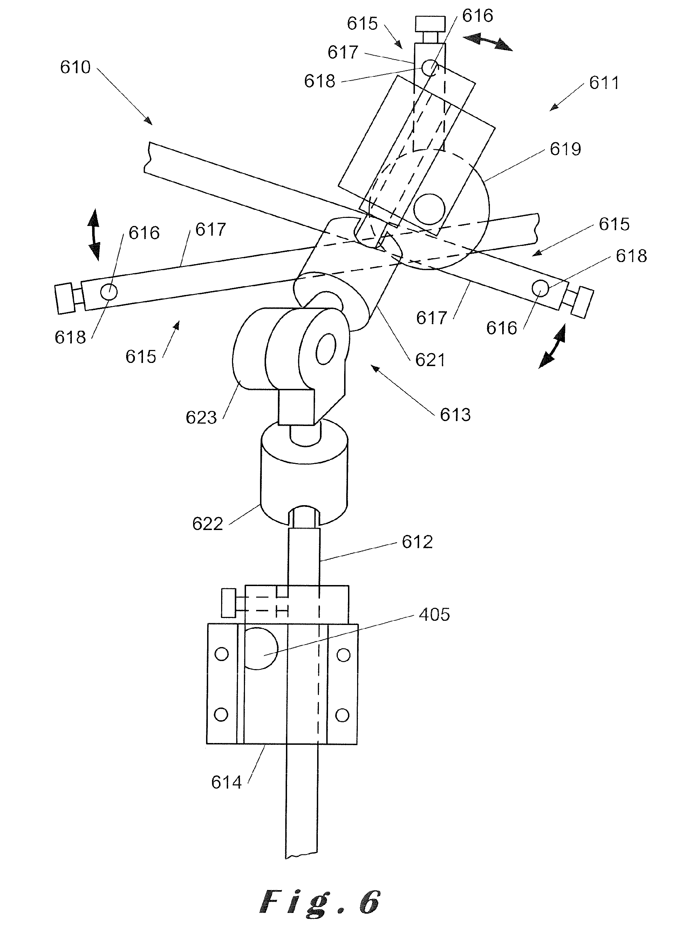

Fig. 6 illustrates how, to check the correct displacement of the upper dental arch 105 during the operation, a device 610 for determining an angle and position of an upper dental arch 105 with respect to a skull reference frame is used in conjunction with the first and second teeth indexing members 405, respectively carrying the first and second moulds.

The device 610 comprises a cranial anchoring section 611, a first calibrated rod 612, an articulated member 613 for coupling said cranial anchoring section 611 to said first calibrated rod 612 and coupling means 614 for releasably locking said first calibrated rod 612 and the first or second teeth indexing member 405 at a fixed angle and position.

The cranial anchoring section 611 comprises three adjustable attachment means 615 for releasably anchoring the device 610 in a stable position to three bolts screwed into the skull 111 at the two brow arches and the hairline. Each one of the three adjustable attachment means 615 comprises an externally threaded spindle 616 with a claw at one end for releasably anchoring said spindle 616 to one of said bolts, and a rod 617 comprising an internally threaded orifice 618 substantially perpendicular to its main axis for receiving said spindle 616, and a means for releasably locking said spindle 616 in position. The cranial anchoring section 611 also comprises coupling means 619 for releasably locking said rods 617 of the adjustable attachment means 614 at fixed angles and positions with respect to each other and to said articulated member 613. The rods 617 of the adjustable attachment means 615 can be simultaneously locked and/or released with a single lever.

The articulated member 613 comprises a first ball joint 621 for coupling the articulated member 613 to the cranial anchoring section 611, a second ball joint 622 for coupling the articulated member 613 to the first calibrated rod 612 and an articulated joint 623 interposed between the first and the second ball joints 621,622. The first and second ball joints 621,622 and the articulated joint 623 can be simultaneously locked at fixed angles or released with a single lever.

In the illustrated embodiment, the first calibrated rod 612, the rods 617 of the adjustable attachment means 614 and the graduated rods of the first and second teeth indexing members 405 have non-rotationally-symmetrical cross-sections over at least part of their lengths, so as to avoid their rotation around their main axis when they are coupled. A preferred cross-section is half-circular, although other forms, such as square or triangular, could also be considered.

Distance graduations are preferably engraved onto the surfaces of the first calibrated rod 612 of the device 610 and the graduated rods of the first and second teeth indexing members 405, so as to more easily register their position when they are coupled.

In order to use the device 610 to check that the displacement of the upper dental arch 105 during the operation corresponds to the displacement of the upper dental arch cast 317 in the 3D orthognatic surgery simulator 510 in one of the simulation methods described above, the first teeth indexing member 405, carrying the first mould, is coupled to the first calibrated rod 612 of the device 610 in a determined position, which is duly registered. The device 610 is then anchored to the skull bolts and adjusted so as to match the first mould with the upper dental arch 105 in the initial position and angle. Once this is correctly matched, all couplings in the device 610 are locked so as to create a stable reference. The device 610 is then released from the skull bolts, and taken away to free access to the face of the patient for the operation. The first teeth indexing member 405 can then be uncoupled from the device 610 and replaced, coupled to the device 610 in the same determined position, by the second teeth indexing member 405, carrying the second mould.

After the upper dental arch 105 has been displaced in the operation, its position can then be checked by anchoring the device 610, coupled to the second teeth indexing member 405, to the skull bolts and checking that the second mould matches the displaced upper dental arch.

Although the present invention has been described with reference to specific exemplary embodiments, it will be evident that various modifications and changes may be made to these embodiments without departing from the broader scope of the invention as set forth in the claims. Accordingly, the description and drawings are to be regarded in an illustrative sense rather than a restrictive sense.

## Claims

1. Method of simulating an orthognatic surgery operation, which operation comprises a surgical intervention on the maxilla (106) of a skull (111) for adjusting the position of the upper dental arch (105) in the skull (111) of a patient, wherein in said method a 3D orthognatic surgery simulator (510) is used, said simulator (510) comprising an upper mounting plate (526) for mounting a model (317), preferably a cast, of said upper dental arch (105), said upper mounting plate (526) of the simulator (510) being mounted on an arm (521);
wherein said simulation method comprises the steps of:
a) mounting said upper dental arch model (317) on the upper mounting plate (526) of the simulator (510);
b) adjusting the angular and translational position of the upper mounting plate (526) so as to place the upper dental arch model (317) in a first position representative of the initial position of said upper dental arch (317) with respect to a fixed point in the skull; and
c) readjusting the angular and translational position of the upper mounting plate (526) so as to simulate the displacement of the upper dental arch (105) caused by the surgical intervention to be applied to the maxilla (106) during the orthognathic surgery;
said method being **characterised in that** the angular and translational position of said upper mounting plate (526) of the simulator (510) is readjusted by rotating said upper mounting plate (526) around at least two, preferably three of three orthogonal axes X,Y and Z with respect to a rotation point (522) fixed to said arm (521), and translating said arm (521) substantially parallely to at least one of the same three orthogonal axes X,Y,Z; said simulator (510) being preferably connectable to a computer for controlling rotational and translational displacements of said upper mounting plate (526) of the simulator (510).

2. Method according to claim 1, further comprising the steps of:
a) taking a first mould of the upper dental arch model (317) in said first position; and
b) taking a second mould of the upper dental arch model (317) after readjusting the angular and translational position of the upper mounting plate (526).

3. Method according to claim 2, wherein the step of taking the first mould comprises the steps of:
a) providing a first teeth indexing member (405) holding a malleable material, preferably acrylic resin, for taking said mould;
b) fixing said first teeth indexing member (405) in a determined position relative to the simulator (510); and
c) pressing said malleable material against the upper dental arch model (317);
and wherein the step of taking the second mould comprises the steps of:
d) providing a second teeth indexing member (405) holding a malleable material, preferably acrylic resin, for taking said mould;
e) fixing said second teeth indexing member (405) in the same determined position relative to the simulator (510); and
f) pressing said malleable material against the upper dental arch model (317).

4. Method according to claim 3, wherein each one of said first and second teeth indexing members (405) comprise a graduated rod, preferably with a half-circular cross-section over at least part of its length, for fixing the teeth indexing member (405) in a determined position and an arch for holding said malleable material.

5. Method according to any one of the previous claims, wherein said orthognathic surgery operation also comprises an adjustment of a lower dental arch (103) with respect to the condyles (107) in the mandible (104) of said skull (111), so as to adjust the occlusion between said upper and lower dental arches (105,103), said method further comprising the steps of :
a) providing a model (318) of the mandible (104) comprising a model (319), preferably a cast, of the lower dental arch (103) ; and
b) simulating in the mandible model (318) the surgical intervention necessary to achieve the desired occlusion; and
c) checking the occlusion between the lower dental arch model (319) and the upper dental arch model (317).

6. Method according to claim 5, wherein said mandible model (318) is a physical model, preferably formed at least partly in resin based on X-ray images of the mandible (104).

7. Method according to claim 6, further comprising the step of hanging said mandible model (318) on an adjustable articulated frame (312) suitable for being mounted in said simulator (510) for reproducing the jaw hinge of the patient.

8. Method according to claim 7, wherein the step of checking the occlusion comprises the following steps:
a) providing a bite mould of the desired occlusion; and
b) placing, in said simulator, said bite mould of the desired occlusion between said upper dental arch model (317) and said lower dental arch model (319).

9. Method according to claim 8, wherein the step of providing a bite mould of the desired occlusion comprises the following steps:
a) providing models (317,319) of the upper and lower dental arches (105,103);
b) placing these models (317,319) of the upper and lower dental arches (105,103) in a desired occlusion with a piece of malleable material between them, so as to form said bite mould of the desired occlusion.

10. Method according to claim 5, wherein said mandible model (318) is a virtual model.

11. Method according to claim 10, further comprising the step of providing a virtual model of the skull (111), comprising at least the maxilla (106) with the upper dental arch (105), wherein the displacement of the upper dental arch model (317) in the simulator is provided to be reflected in said virtual model of the skull (111).

12. Method according to claim 11, further comprising the step of providing a CT-scan of the skull (111), wherein said virtual model of the skull (111) is based on said CT-scan of the skull (111).

13. Method according to claim 12, wherein the CT-scan of the skull (111) is a CT-scan taken with a piece of malleable material, comprising radio-opaque references, held between the upper and lower dental arches (105,104), so as to form a bite mould of the initial occlusion, and wherein the method further comprises the following steps:
a) providing models (317,319) of the upper and lower dental arches (105,103);
b) placing these models (317,319) of the upper and lower dental arches (105,103) in a desired occlusion with a piece of malleable material, also comprising radio-opaque references, between them, so as to form a bite mould of the desired occlusion; and
c) taking a CT-scan of said models (317,319) of the upper and lower dental arches (317,319) in the desired occlusion, with the bite mould of the desired occlusion, for preparing the step of simulating the surgical intervention in the virtual mandible model.

14. Method according to claim 13, further comprising a step of taking a CT-scan of said models of the upper and lower dental arch in the initial occlusion, with the bite mould of the initial occlusion.

15. A device (610) for determining an angle and a position of an upper dental arch (105) with respect to a skull (111), comprising:
a) a cranial anchoring section (611) for releasably anchoring the device (610) in a stable position to a plurality of bolts, preferably three, screwed into the skull (111), preferably at the two brow arches and the hairline;
b) a first calibrated rod (612), preferably having a half-circular cross-section over at least part of its length; and
c) coupling means (614) for releasably locking said first calibrated rod (612) and a teeth indexing member (405) at a fixed angle and position;
wherein the device (610) is **characterised in that** it also comprises:
d) an articulated member (613) for coupling said cranial anchoring section (611) to said first calibrated rod (612) and comprising a first ball joint (621) for coupling the articulated member (613) to the cranial anchoring section (611), a second ball joint (622) for coupling the articulated member (613) to the first calibrated rod (612) and an articulated joint (623) interposed between the first and the second ball joints (621,622), wherein the first and second ball joints (621,622) and the articulated joint (623) are provided to be releasably locked at fixed angles.

16. Device (610) according to claim 15, wherein the first and second ball joints (621,622) and the articulated joint (623) of said articulated member (613) are provided to be simultaneously locked and/or released with a single lever.

17. Device (610) according to one of claims 15 or 16, wherein said cranial anchoring section (611) comprises at least three adjustable attachment means (615), each one of them comprising:
a) an externally threaded spindle (616) with a claw at one end for releasably anchoring said spindle (616) to one of said bolts; and
b) a rod (617), preferably having a semicircular cross-section over at least part of its length, and comprising an internally threaded orifice (618) substantially perpendicular to its main axis for receiving said spindle (616), and a means for releasably locking said spindle (616) in position; and
wherein said cranial anchoring section (611) also comprises coupling means (619) for releasably locking said rods (617) of the adjustable attachment means (615) at fixed angles and positions with respect to each other and to the first ball joint (621) of said articulated member (613).

18. Device (610) according to claim 17, wherein said rods (617) of the adjustable attachment means (615) are provided to be simultaneously locked and/or released with a single lever.

19. A method of using a device (610) according to one of claims 15 to 18 for checking an upper dental arch (105) adjustment from an initial to a desired position and angle with respect to the skull (111) comprising the steps of:
a) coupling to said device (610) in a determined position a first teeth indexing member (405) carrying a mould of the upper dental arch (105) in the initial position and angle;
b) anchoring said device (610) to the skull bolts and adjusting it so as to match the upper dental arch mould with the upper dental arch (105) in the initial position and angle;
c) locking all couplings in the device (610);
d) releasing said device (610) from the skull bolts;
e) uncoupling said first teeth indexing member (405) and coupling to said device (610), in the same determined position, a second teeth indexing member (405) carrying a mould of the upper dental arch in the desired position and angle;
f) anchoring the device (610) to the skull bolts after the upper dental arch (105) has been displaced and checking that the mould of the upper dental arch (105) in said desired position and angle matches the displaced upper dental arch (105).

20. A method according to claim 19, wherein said first and second teeth indexing members (405) are provided using the simulation method of claim 3.
